(19) Europäisches Patentamt
European Patent Office
Office européen des brevets

(11) Numéro de publication: **0 296 046 B1**

(12) **FASCICULE DE BREVET EUROPEEN**

(45) Date de publication du fascicule du brevet: **07.11.90**

(51) Int. Cl.5: **C07C 215/02,** C07C 219/02, C10M 133/08

(21) Numéro de dépôt: **88401453.1**

(22) Date de dépôt: **13.06.88**

(54) **Amino-alcools polyfluorés et leurs esters, preparation de ces composés et leur utilisation comme additifs pour lubrifiants.**

(30) Priorité: **19.06.87 FR 8708662**

(43) Date de publication de la demande: **21.12.88 Bulletin 88/51**

(45) Mention de la délivrance du brevet: **07.11.90 Bulletin 90/45**

(84) Etats contractants désignés: **AT BE CH DE ES FR GB GR IT LI LU NL SE**

(56) Documents cités:
**WO-A-83/02622**
**US-A- 3 873 619**
**US-A- 4 059 629**

(73) Titulaire: **ATOCHEM, 4 & 8, Cours Michelet La Défense 10, F-92800 Puteaux(FR)**

(72) Inventeur: **Durual, Pierre, Les Essarts 2 481 Chemin de la Rossignole, F-69390 Vernaison(FR)**
Inventeur: **Hermant, Marc, 10 ter, Rue Lamartine, F-95240 Cormeilles-en-Parisis(FR)**
Inventeur: **Laviron, Charles, 27 A, Rue Soeur Bouvier, F-69005 Lyon(FR)**

(74) Mandataire: **Leboulenger, Jean et al, ATOCHEM Département Propriété Industrielle, F-92091 Paris la Défense 10 Cédex 42(FR)**

**EP 0 296 046 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne le domaine des produits fluorés et celui des lubrifiants. Elle a plus particulièrement pour objet de nouveaux composés polyfluorés utilisables comme additifs anti-usure pour lubrifiants.

L'application de certains dérivés organo-fluorés comme additifs à des compositions lubrifiantes est connue ; par exemple l'application des sels d'amines aliphatiques et d'acides monocarboxyliques perhalogénés a été décrite dans le brevet US 3 565 926 de même que l'application des dérivés obtenus par réaction d'une amine aromatique et d'un composé organique fluoré choisi parmi les monoacides carboxyliques fluorés a été divulguée dans le brevet FR 2 026 493. Cependant ces dérivés carboxyliques présentent l'inconvénient de perdre leur efficacité anti-usure en présence d'additifs classiques tels que les additifs dispersants-détergents, soit par suite d'interactions physico-chimiques qui empêchent leur absorption au niveau des surfaces à lubrifier, soit à cause d'interactions chimiques, en particulier lorsque les additifs dispersants-détergents sont des sels de métaux alcalino-terreux neutres ou surbasés.

Selon le brevet FR 2 520 377, on a obtenu des compositions lubrifiantes possédant des propriétés anti-usure et un pouvoir réducteur du frottement remarquables en utilisant comme additif organo-fluoré des amines ou amino-alcools à chaîne polyfluorée et, plus particulièrement des amino-alcools qui peuvent être représentés par la formule :

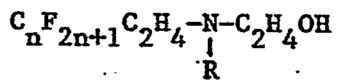 (I)

où n est un nombre entier compris entre 2 et 20 et R est un atome d'hydrogène ou un radical hydroxyéthyle. Ainsi qu'il est indiqué à la page 4 de ce brevet, ces amino-alcools obtenus par condensation d'un iodure de perfluoroalkyl-2 éthyle avec l'éthanolamine ou la diéthanolamine se présentent industriellement sous forme de mélanges contenant une proportion non négligeable (le plus souvent, de 30 à 50 % en poids) d'amino-alcools insaturés de formule :

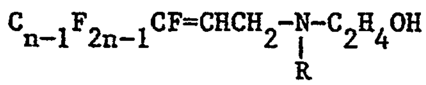 (II)

qu'il n'est pas économique de séparer. Ces amino-alcools insaturés s'obtiennent également par réaction d'une oléfine $C_nF_{2n+1}$-CH=$CH_2$ avec un amino-alcool R-NH-$CH_2CH_2OH$ (brevet US 3 535 381).

Il a maintenant été trouvé que les produits fluorés obtenus par hydrogénation des composés de formule (II) ou des mélanges industriels les contenant présentent une efficacité anti-usure sensiblement meilleure. Il a également été constaté que les dérivés obtenus par réaction avec un époxyde et/ou estérification des produits hydrogénés présentent eux aussi une excellente efficacité anti-usure.

La présente invention a donc pour objet les composés polyfluorés de formule générale :

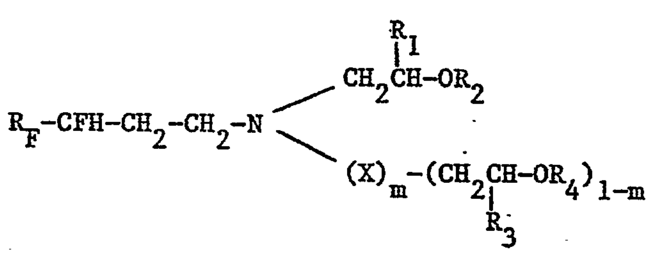 (III)

et leurs mélanges entre eux et/ou avec jusqu'à environ 80 % de composés polyfluorés de formule :

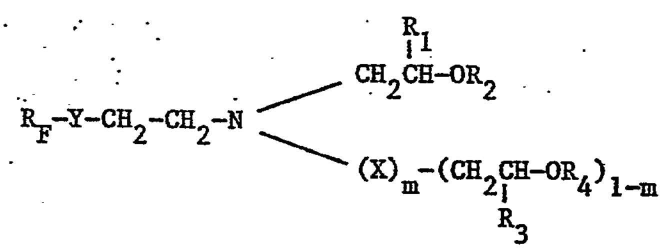 (IV)

formules dans lesquelles :

$R_F$ représente un radical perfluoroalkyle, linéaire ou ramifié, contenant de 1 à 19 atomes de carbone, de préférence de 5 à 15,
$R_1$ et $R_3$ identiques ou différents, représentent chacun un atome d'hydrogène, un radical alkyle contenant de 1 à 20 atomes de carbone, un radical cycloalkyle à 5 ou 6 atomes de carbone ou un radical aryle éventuellement substitué,
$R_2$ et $R_4$ identiques ou différents, représentent chacun un atome d'hydrogène ou le reste acyle d'un acide carboxylique aliphatique, cycloaliphatique ou aromatique,
m est égal à zéro ou un,
X représente un atome d'hydrogène ou le groupement hydroxy-2 phényl-1 éthyle, et
Y représente un radical $CF_2$ ou $CH_2$

Les composés et mélanges selon l'invention peuvent être préparés à partir des amino-alcools de formule :

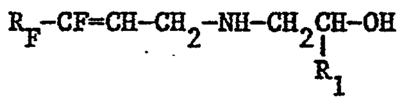

(V)

ou de mélanges de ces amino-alcools entre eux et/ou avec jusqu'à environ 70 % d'amino-alcools saturés de formule :

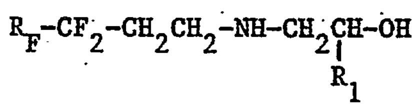

(IV-a)

par hydrogénation, puis éventuellement réaction avec un époxyde et/ou estérification.

L'hydrogénation qui conduit aux composés polyfluorés de formule :

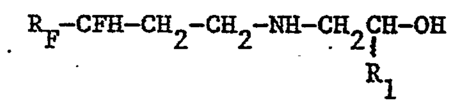

(III-a)

(formule III avec m=1 et X=$R_2$=H) ou aux mélanges de ces composés III-a avec les amino-alcools de formule :

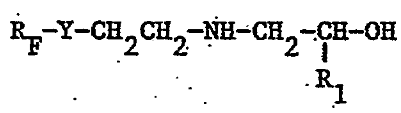

(IV-b)

pour être effectuée suivant les méthodes d'hydrogénation connues en utilisant un catalyseur choisi parmi les métaux du groupe 8 et leurs oxydes, de préférence le palladium sur charbon ou le nickel de Raney. On peut opérer en présence ou non d'un solvant, sous une pression d'hydrogène allant de la pression atmosphérique jusqu'à 200 bars (de préférence, entre 5 et 100 bars) et à une température pouvant aller de 25 à 250°C (de préférence, entre 50 et 150°C). Pour faciliter la récupération du catalyseur, il est avantageux d'opérer au sein d'un solvant, de préférence un alcool et plus particulièrement le méthanol ou l'éthanol dont les températures d'ébullition facilitent l'élimination.

On peut obtenir les composés fluorés de formule:

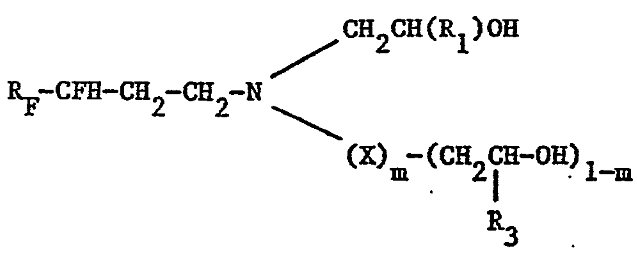

(III-b)

(formule III avec m = 0 ou 1 et X = hydroxy-2 phényle-1 éthyle) et leurs mélanges avec les amino-diols de formule :

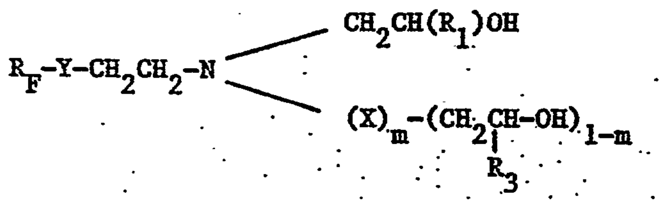

(IV-c)

en faisant réagir un époxyde sur les amino-alcools III-a ou sur les mélanges d'amino-alcools III-a + IV-b.

Comme exemples non limitatifs d'époxydes, on peut mentionner plus spécialement l'oxyde d'éthylène, l'oxyde de propylène, l'époxy-1,2 butane, l'époxy-1,2 hexane, l'époxy-1,2 dodécane, l'époxy-1,2 octadécane et l'oxyde de styrène. La réaction avec un époxyde peut être réalisée de différentes façons selon la nature de l'époxyde employé. Si l'on met en oeuvre un époxyde normalement gazeux, on opère de préférence par barbotage ou en autoclave, alors qu'avec un époxyde liquide on peut opérer par simple chauffage du mélange de l'époxyde et du ou des amino-alcools.

Les produits selon l'invention dans lesquels $R_2$ et/ou $R_4$ représentent un reste acyle peuvent être préparés par estérification des amino-diols III-b (ou mélanges III-b + IV-c) au moyen d'un acide carboxylique ou d'un dérivé de formule :

R - CO - Z(VI)

où Z représente le groupement OH, un atome de chlore ou un groupe alcoxy contenant de 1 à 5 atomes de carbone, et R représente un radical aliphatique, linéaire ou ramifié, saturé ou non, contenant de 1 à 30 et de préférence 4 à 22 atomes de carbone, un radical cycloaliphatique ou un radical aromatique. Cette réaction peut être réalisée entre 0 et 100°C.

Quand on met en oeuvre un acide (Z=OH), on opère en présence d'un capteur d'eau tel que l'acide sulfurique ou un tamis moléculaire, L'eau formée peut être éliminée par distillation azéotropique à l'aide d'un solvant inerte, de préférence un solvant aromatique tel que, par exemple, le benzène, le toluène ou le xylène.

Si l'on utilise un ester d'acide carboxylique (Z=alcoxy), on opère en présence d'un catalyseur de transestérification, par exemple l'acide sulfurique, l'acide p-toluènesulfonique ou un alcoolate d'aluminium. On peut employer l'ester R COZ en excès comme solvant réactionnel.

Quand l'esterification est effectuée au moyen d'un chlorure d'acide (Z=Cl) on opère en présence d'un capteur d'hydracide, tel que les amines tertiaires contenant 3 à 20 atomes de carbone et choisi de préférence parmi la triméthylamine, la triéthylamine, la tripropylamine, la tributylamine, la tripentylamine et la pyridine. Ce type d'estérification est généralement réalisé au sein d'un solvant constitué par un éther aliphatique (éther éthylique, propylique, isopropylique, butylique, isobutylique ou amylique, méthyl tert-butyl éther, méthyl tert-amyl éther) ou un hydrocarbure aliphatique halogéné comme, par exemple, le chlorure de méthylène et le chloroforme.

Comme exemples de chlorures d'acide utilisables, on peut citer plus particulièrement les chlorures de butyle, de caproyle, de caprylyle, d'isovaléryle, de lauroyle, de linoléyle, d'heptanolyle, d'oléyle, de palmitoyle, de pélargonyle, de phénylacétyle, de pivaloyle, de stéaroyle, d'undécènoyle, de benzoyle, de méthyl-2 benzoyle, de tert-butyl-4 benzoyle ou de cinnamoyle.

Parmi les produits polyfluorés selon l'invention, on préfère tout particulièrement ceux dans lesquels :

- m est égal à 1 et $R_1$ et $R_2$ sont des atomes d'hydrogène ;
- m est égal à 0, $R_1$, $R_2$ et $R_4$ sont des atomes d'hydrogène et $R_3$ est un radical éthyle ; ou
- m est égal à 0, $R_1$, $R_2$ et $R_3$ sont des atomes d'hydrogène et $R_4$ est un radical benzoyle.

La quantité de produit polyfluoré selon l'invention à ajouter à une huile lubrifiante pour obtenir une efficacité anti-usure est d'au moins 0,01 % par rapport au poids de l'huile et est de préférence comprise entre 0,02 et 0,5 %.

L'huile lubrifiante peut être une huile minérale, un hydrocarbure synthétique, une huile synthétique appartenant aux différentes familles suivantes : glycols, éthers de glycols, esters de glycols, les polyoxyalkylène glycols, leurs éthers et leurs esters, les esters d'acides monocarboxyliques ou polycarboxyliques et de monoalcools ou polyalcools, cette énumération n'étant pas limitative.

Lorsqu'on utilise comme bases lubrifiantes, les coupes pétrolières destinées à la fabrication des huiles moteurs, telles que les bases "Neutral Solvent", on associe avantageusement aux dérivés fluoro-organiques de l'invention des additifs dispersants-détergents traditionnels tels que les alkylaryl sulfonates et les alkylphénates de calcium ou de baryum, ou des dispersants "sans cendres" comme les dérivés succiniques. Les additifs dispersants-détergents favorisent la solubilisation des additifs fluorés dans l'huile sans porter atteinte aux propriétés anti-usure de ces derniers et sans perdre leur propre pouvoir.

L'addition de dérivés fluorés selon l'invention aux huiles formulées contenant déjà des additifs tels que les alkyldithiophosphates de zinc apporte une amélioration sensible du pouvoir anti-usure et une augmentation de la capacité de charge de ces huiles sans perturbation des propriétés apportées par les autres additifs : dispersivité, détergence, pouvoir anti-corrosion, par exemple.

Le remplacement dans les formulations d'huiles pour moteurs à combustion interne ou pour systèmes hydraulique de transmission d'énergie, de tout ou partie du dithiophosphate de zinc utilisé comme additif anti-usure par 0,02 à 0,2 % de composés organofluorés selon l'invention permet d'atteindre un niveau de protection contre l'usure égal ou supérieur à celui obtenu avec cet additif traditionnel.

Les additifs fluorés selon l'invention peuvent donc être utilisés soit en remplacement des alkyldithiophosphates de zinc, soit en suradditivation dans les huiles lubrifiantes pour moteurs à essence ou moteurs diesel , ainsi que dans les huiles hydrauliques.

Les exemples et les tests suivants illustrent l'invention sans la limiter. Les pourcentages s'entendent en poids, sauf mention contraire.

**EXEMPLE 1**

a/ Dans un réacteur de 4 litres, équipé d'une agitation, d'un réfrigérant et d'un thermomètre, on charge 1410 g de (perfluorooctyl)-éthylène $C_8F_{17}$-CH=$CH_2$ à 95 %, 0,96 litre de n-pentanol, 252 g de bicarbonate de sodium et 732 g de monoéthanolamine, puis on chauffe le mélange à reflux (117 à 121°C) pendant 7 heures.

Le mélange réactionnel marron est ensuite refroidi à 35-40°C, puis lavé 5 fois par 1,5 litre d'eau ) 35-40°C. La phase organique ainsi obtenue (2155 g) est ensuite étêtée sur évaporateur à film à 85°C, sous 667 Pa, au débit de 0,42 l/h pour éliminer l'eau résiduelle, une partie du n-pentanol et le (perfluorooctyl)-éthylène n'ayant pas réagi. On obtient ainsi 1306 g d'un mélange contenant 78,5 % de produit fluoré et 21,5 % de n-pentanol.

Ce mélange est ensuite étêté à 50 % sur une colonne à distiller sous 1333 Pa, puis le pied de distillation (1242 g) est distillé sur évaporateur à film à 167°C sous 67 à 133 Pa, au débit de 0,15 1/h. On recueille ainsi en tête 1180 g d'une huile jaune dont la composition molaire, déterminée par RMN, est la suivante :

$C_7F_{15}$-CF=CH-$CH_2$-NH-$CH_2CH_2OH$ : 88,5 %
$C_8F_{17}$-$CH_2CH_2$-NH-$CH_2CH_2OH$ : 11,5 %

b/ Dans un autoclave en acier inoxydable de 2 litres, muni d'un système d'agitation à entraînement magnétique, on charge 1000 g de l'huile obtenue ci-dessus, 0,6 litre d'éthanol à 99 % et 16 g d'une suspension de nickel de Raney à environ 60 % dans l'éthanol à 99 %, puis on effectue 3 purges à l'azote sous 20 bars et ensuite 3 purges à l'hydrogène sous 20 bars. On hydrogène alors pendant 12 heures à 70-75°C, en agitant à 1500 tr/min et en maintenant la pression d'hydrogène entre 8 et 11 bars.

Après refroidissement et purge de l'autoclave, on filtre le catalyseur et élimine l'éthanol par distillation. On obtient ainsi 1025 g d'un solide cireux jaune clair dont l'analyse CPV fournit la composition molaire suivante :

$C_7F_{15}$-CFH-$CH_2CH_2$-NH-$CH_2CH_2OH$ : 64,1 %
$C_7F_{15}$-$CH_2CH_2CH_2$-NH-$CH_2CH_2OH$ : 24,9 %
$C_8F_{17}$-$CH_2CH_2$-NH-$CH_2CH_2OH$ : 11 %

Par purification sur colonne de silice, on isole le produit principal :
$C_7F_{15}$-CFH-$CH_2CH_2$-NH-$CH_2$-$CH_2OH$
qui présente les caractéristiques suivantes :

Point de fusion : 54-55°C
Ebullition : 260°C sous $10^5$ Pa
RMN$^{13}$C ($CDCl_3$, TMS) : δ (ppm)
- <u>C</u>FH- : 86,9
- <u>C</u>$H_2OH$ : 60,7
- <u>C</u>$H_2CH_2OH$ : 51,0
- <u>C</u>$H_2$-NH- : 44,0
- CFH-<u>C</u>$H_2$- : 27,6

**EXEMPLE 2**

Dans un erlen de 0,1 litre, surmonté d'un réfrigérant et agité par barreau magnétique, on place 25,35 g du solide cireux obtenu à l'exemple 1-b et 4,45 g d'époxy-1,2 butane, puis on chauffe sous agitation pendant 18 heures à 65°C.

On laisse ensuite évaporer l'excès d'époxyde à 65°C sous pression ambiante, puis on élimine les dernières traces sous vide. On obtient ainsi 28 g d'un liquide brun constitué par les diols suivants:

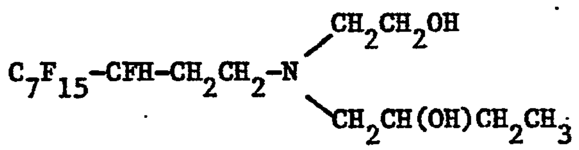
: 64,1 % molaire

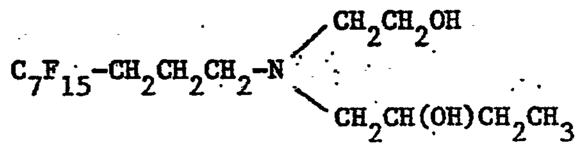
: 24,9 % molaire

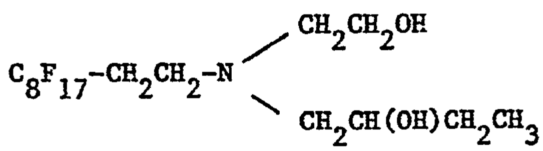
: 11 % molaire

Les caractéristiques RMN13C ($CDCl_3$) du diol principal sont les suivantes:

| | δ (ppm) |
|---|---|
| $-\underline{C}H(OH)-$ | 69,6 |
| $-\underline{C}H_2CH(OH)-$ | 60,4 |
| $-\underline{C}H_2OH$ | 59,3 |
| $-\underline{C}H_2CH_2OH$ | 56,1 |
| $-\underline{C}H_2-N<$ | 46,2 |
| $-CFH-\underline{C}H_2-$ | 28,2 |
| $-\underline{C}H_2-CH_3$ | 27,5 |
| $-\underline{C}H_3$ | 9,6 |

## EXEMPLE 3

Dans un réacteur de 0,25 litre, équipé d'une agitation, d'un thermomètre et d'un réfrigérant, on place 150 g du solide cireux obtenu à l'exemple 1-b et 36,6 g d'époxy-styrène, puis on chauffe pendant 6 heures à 110°C.

On recueille ainsi 171 g d'une huile marron dont les constituants principaux présentent les caractéristiques RMN13C ($CDCl_3$) suivantes :

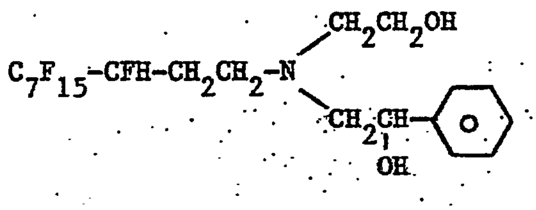

| δ (ppm) | |
|---|---|
| cycle $C_6$ | 142,1 - 127,4 - 127,2 et 125 |
| $-\underline{C}H(OH)-$ | 70,1 |
| $-\underline{C}H_2-CH(OH)-$ | 62,4 |
| $-\underline{C}H_2OH$ | 58,6 |
| $-\underline{C}H_2CH_2OH$ | 55,7 |
| $-\underline{C}H_2-N<$ | 45,4 |
| $-CFH-\underline{C}H_2-$ | 27,8 |

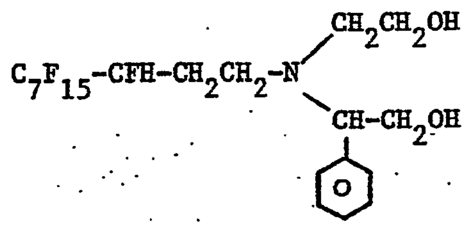

| δ (ppm) | |
|---|---|
| cycle $C_6$ | 142 - 127,4 - 127,2 et 125 |
| $-\underline{C}H-Ø$ | 65,9 |
| $>CH-\underline{C}H_2OH$ | 60,9 |
| $-CH_2-\underline{C}H_2OH$ | 58,3 |
| $-\underline{C}H_2CH_2OH$ | 55,7 |
| $-\underline{C}H_2-N<$ | 43,2 |
| $-CFH-\underline{C}H_2-$ | 29,3 |

**EXEMPLE 4**

a/ Dans un autoclave en acier inoxydable de 4 litres, muni d'un système d'agitation à entraînement magnétique, on charge 2000 g d'un mélange d'amino-alcools fluorés $C_8F_{17}$-$CH_2CH_2$-NH-$CH_2CH_2OH$ (67 % molaire) et $C_7F_{15}$-CF=CH-$CH_2$-NH-$CH_2CH_2OH$ (33 % molaire), puis 1,2 litre d'éthanol à 99 % et 32 g d'une suspension de nickel de Raney à environ 60 % dans l'éthanol 99 %. L'autoclave est ensuite purgé 3 fois à l'azote sous 30 bars, puis 3 fois à l'hydrogène sous 30 bars.

On hydrogène ensuite pendant 6 heures et 45 minutes à 70°C, en agitant à 2000 tr/min et en maintenant la pression à 20 bars. Après refroidissement, détente et purge de l'autoclave, on filtre le catalyseur et évapore l'éthanol.

On obtient ainsi 1940 g d'un solide jaune clair qui fond à 51°C et dont l'analyse CPV donne la composition suivante :

$C_8F_{17}$-$CH_2CH_2$-NH-$CH_2CH_2OH$ : 65,6 %
$C_7F_{15}$-CFH-$CH_2CH_2$-NH-$CH_2CH_2OH$ : 25,4 %
$C_7F_{15}$-$CH_2CH_2CH_2$-NH-$CH_2CH_2OH$ : 8,9 %

b/ En opérant dans les mêmes conditions que ci-dessus, mais à 150°C, l'hydrogénation n'a duré qu'une heure et on a obtenu des résultats similaires.

c/ En opérant dans un autoclave de 0,2 litre et sur 115 g du même mélange d'amino-alcools fluorés, sans solvant, en présence de 0,5 g de catalyseur à 5 % de palladium sur charbon, l'hydrogénation a été réalisée en 4 heures sous 5 bars à 50°C et on a obtenu des résultats identiques.

d/ Si, dans le même autoclave, on opère sur les mêmes quantités d'amino-alcools et de catalyseur au palladium, mais en présence de 75 g d'éthanol comme solvent, l'hydrogénation est réalisée en une heure sous 50 bars, la température variant de 55 à 65°C. Les résultats sont encore identiques. Il en est de même lorsqu'on remplace l'éthanol par le méthanol.

**EXEMPLE 5**

En opérant comme à l'exemple 4-a, on hydrogène pendant 9 heures à 80°C 2000 g d'un mélange industriel d'amino-alcools fluorés de formules :
$C_nF_{2n+1}$-$CH_2CH_2$-NH-$CH_2CH_2OH$(67 % molaire)
$C_{n-1}F_{2n-1}$-CF=CH-$CH_2$-NH-$CH_2CH_2OH$(33 % molaire)
dont la répartition pondérale des chaînes fluorées est la suivante:

| n | % |
|---|---|
| 6 | 55,7 |
| 8 | 27,2 |
| 10 | 10,15 |
| 12 | 3,9 |
| ≥ 14 | 2,9 |

Après filtration du catalyseur et évaporation de l'éthanol, on obtient 1990 g d'un produit jaune clair, mi-liquide mi-solide (entièrement liquide à 45°C), dont l'analyse CPV donne les résultats suivants :

$C_nF_{2n+1}$-$CH_2CH_2$-NH-$CH_2CH_2OH$ : 69,3 %
$C_{n-1}F_{2n-1}$-CFH-$CH_2CH_2$-NH-$CH_2CH_2OH$ : 18,6 %
$C_{n-1}F_{2n-1}$-$CH_2CH_2CH_2$-NH-$CH_2CH_2OH$ : 9,8 %

**EXEMPLE 6**

Dans un réacteur de 2 litres, équipé d'un agitateur, d'un thermomètre et d'un réfrigérant, on charge 1200 g du produit obtenu à l'exemple 5 et 309 g d'oxyde de styrène, puis on chauffe sous agitation jusqu'à 118°C et arrête ensuite de chauffer. La température monte alors d'elle-même jusqu'à 125°C en 5 minutes. On refroidit jusqu'à 108°C en 15 minutes, puis on reprend le chauffage et on maintient à 118-120°C pendant encore 2 heures et quart.

On ajoute alors 4,8 g d'oxyde de styrène et chauffe à 110-115°C pendant encore 3 heures. On obtient ainsi 1508 g d'un produit visqueux jaune orangé dont l'analyse CPV montre qu'il ne contient que 0,05 % d'oxyde de styrène libre.

**EXEMPLE 7**

a/ Dans un réacteur de 2 litres, équipé d'une arrivée de gaz avec tube plongeur précédé d'un compte-bulles, d'un système d'agitation, d'un thermomètre et d'une sortie de gaz avec compte-bulles, on place 1250 g du produit obtenu à l'exemple 5, puis on chauffe sous agitation jusqu'à 58°C et balaie le circuit de gaz par de l'azote.

On introduit ensuite de l'oxyde d'éthylène à un débit tel qu'on n'observe qu'un léger barbotage dans le compte-bulles de sortie. On introduit ainsi 189 g d'oxyde d'éthylène en 11 heures et quart, en maintenant la température à 60°C.

Après refroidissement, on recueille 1402 g d'un mélange de diols fluorés, sous forme d'une huile jaune avec du solide jaune clair (entièrement liquide à 45-50°C). L'analyse CPV montre que ce produit ne contient pas de mono-alcools.

b/ Dans un ballon de 10 litres équipé d'un réfrigérant, d'une ampoule de coulée, d'un thermomètre, d'une agitation et refroidi par un bain de glace, on charge 940 g du mélange de diols obtenu ci-dessus, 197 g de triéthylamine et 4,5 litres de diisopropyl éther.

L'agitation étant lancée à 200 tr/min, on ajoute en 40 minutes une solution de 260 g de chlorure de benzoyle dans 360 ml de diisopropyl éther, tout en maintenant la température à 18-22°C. L'addition terminée, on rince l'ampoule de coulée avec 140 ml de diisopropyl éther et on retire la bain de galce. On laisse alors sous agitation pendant une heure à température ambiante, puis on chauffe à 60°C pendant 4 heures.

On refroidit ensuite jusqu'à environ 30°C, puis filtre le solide (chlorhydrate de triéthylamine) et évapore le diisopropyl éther. On obtient ainsi 1089 g d'une huile jaune foncé constituée principalement par les monobenzoates des diols fluorés de départ.

**TESTS ANTI-USURE**

I - Le pouvoir anti-usure de compositions lubrifiantes contenant comme huile de base l'huile minérale 200 Neutral Solvent et comme additif un produit fluoré selon l'invention a été déterminé à l'aide de la machine 4 billes EP et SHELL dont la description figure dans "Annual Book of ASTM Standards", Part 24 (1979), pages 680 à 688.

Le test consiste à faire tourner une bille de 12 mm de diamètre avec une vitesse de rotation de 1500 tr/min sur trois autres billes maintenues immobiles et couvertes de lubrifiant à étudier. Une charge de 40 ou 70 daN est appliquée par un système de levier qui pousse les trois billes fixes vers la bille supérieure placée dans un mandrin.

L'efficacité anti-usure d'un lubrifiant est déterminée par la valeur moyenne des diamètres des empreintes d'usure sur les trois billes fixes, après une heure de fonctionnement.

Le tableau I suivant rassemble les résultats obtenus avec différents additifs fluorés selon l'invention, identifiés sous la forme Hx où x correspond au numéro de l'exemple décrivant la préparation de l'additif fluoré. Dans la seconde colonne, est indiquée la proportion pondérale d'additif fluoré incorporé dans l'huile de base.

A titre comparatif sont également indiqués les résultats obtenus avec les homologues non hydrogénés suivants :

I 1 : huile de l'exemple 1-a

I 2 : produit obtenu en faisant réagir 3 g d'époxy-2 butane avec 15 g de l'huile de l'exemple 1-a, dans les mêmes conditions opératoires qu'à l'exemple 2

I 3 : idem, mais en remplaçant l'époxy-butane par 3,6 g d'époxystyrène et en chauffant 5 heures à 110°C

I 4 : mélange d'amino-alcools fluorés, utilisé comme produit de départ à l'exemple 4-a

I 5 : mélange industriel d'amino-alcools fluorés, utilisé comme produit de départ à l'exemple 5

I 6 : produit obtenu par action de l'oxyde de styrène (144,3 g) sur le mélange I5 (570 g) dans les mêmes conditions qu'à l'exemple 6

I 7 : produit obtenu par action de 5,52 g de chlorure de benzoyle sur 20,4 g du mélange de diols obtenu comme à l'exemple 7-a, mais à partir du produit I5.

TABLEAU I

| Additif fluoré | Proportion % poids | Diamètre d'empreinte en mm pour une charge appliquée de 40 daN | 70 daN |
|---|---|---|---|
| Néant (témoin) | | 1,44 | 2,37 |
| I 1<br>H 1-b | 0,05<br>0,05 | 0,85<br>0,48 | 0,90<br>0,59 |
| I 2<br>H 2 | 0,1<br>0,1 | 0,76<br>0,59 | 1<br>0,76 |
| I 3<br>H 3 | 0,1<br>0,1 | 0,71<br>0,55 | 0,96<br>0,78 |
| I 4<br>H 4-a | 0,1<br>0,1 | 0,74<br>0,51 | 0,98<br>0,75 |
| I 5<br>H 5 | 0,05<br>0,05 | 0,75<br>0,55 | 0,81<br>0,66 |
| I 6<br>H 6 | 0,1<br>0,1 | 0,68<br>0,40 | 0,9<br>0,52 |
| I 7<br>H 7-b | 0,1<br>0,1 | 0,66<br>0,44 | 0,8<br>0,55 |

L'examen de ces résultats montre que les produits H1 à H7 selon l'invention présentent une efficacité anti-usure nettement améliorée par rapport à leurs homologues I1 à I7 qui n'ont pas subi l'hydrogénation.

II - L'additif fluoré H6 selon l'invention a été testé comparativement à trois additifs anti-usure du type dithiophosphate de zinc actuellement utilisés dans les huiles hydrauliques, à savoir :

DTPZ A : dithiophosphate de zinc dérivé d'alcool secondaire en $C_6$

DTPZ B : dithiophosphate de zinc dérivé d'alcool primaire en $C_8$

DTPZ C : dithiophosphate de zinc dérivé d'un mélange d'alcools primaires et secondaires.

Les additifs ont été mis en solution à diverses concentrations dans une huile paraffinique 200N et chaque solution a ensuite été soumise au test d'usure à la machine 4 billes EP sous une charge de 40 daN pendant 1 et 2 heures, la température étant maintenue à 120°C durant le test.

Les résultats sont rassemblés dans le tableau II suivant. Leur examen montre que le diamètre d'usure pour l'additif H6 selon l'invention est toujours moindre que ceux correspondant aux dithiophosphates de zinc qui sont pourtant utilisés à des teneurs dix fois plus élevées.

TABLEAU II

| ADDITIFS | CONCENTRATION (% poids) | DIAMETRE D'EMPREINTE EN MM 1 heure | 2 heures |
|---|---|---|---|
| NEANT (témoin) | | 1,39 | 1,55 |
| DTPZ A | 0,2 | 0,87 | 1,27 |
| | 0,5 | 0,61 | 0,65 |
| | 1 | 0,59 | 0,66 |
| DTPZ B | 0,2 | 1 | 1,12 |
| | 0,5 | 0,92 | 1,01 |
| | 1 | 0,60 | 0,66 |
| DTPZ C | 0,2 | 0,72 | 0,74 |
| | 0,5 | 0,59 | 0,66 |
| | 1 | 0,66 | 0,79 |
| H6 | 0,02 | 0,44 | 0,51 |
| | 0,05 | 0,46 | 0,50 |
| | 0,1 | 0,44 | 0,53 |

III - Une huile hydraulique commerciale ISO VG46 a été suradditivitée avec l'additif fluoré H6 selon l'invention utilisé à différentes concentrations. Le pourvoir anti-usure a été testé sur la machine 4 billes EP sous une charge de 40 daN à 120°C pendant 1 et 2 heures.

Le tableau III suivant rassemble les résultats obtenus.

TABLEAU III

| TENEUR EN ADDITIF FLUORE H6 (% poids) | DIAMETRE D'EMPREINTE EN MM | |
|---|---|---|
| | 1 heure | 2 heures |
| 0 (témoin) | 0,62 | 0,72 |
| 0,02 | 0,57 | 0,63 |
| 0,05 | 0,46 | 0,49 |
| 0,1 | 0,46 | 0,49 |

**Revendications pour les Etats contractants: AT, BE, CH, DE, FR, GB, GR, IT, LI, LU, NL, SE**

1. Composés polyfluorés, caractérisés en ce qu'ils répondent à la formule générale :

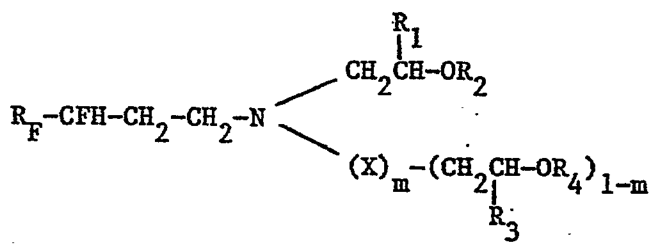

(III)

dans laquelle :
$R_F$ représente un radical perfluoroalkyle, linéaire ou ramifié, contenant de 1 à 19 atomes de carbone,
$R_1$ et $R_3$ identiques ou différents, représentent chacun un atome d'hydrogène, un radical alkyle contenant de 1 à 20 atomes de carbone, un radical cycloalkyle à 5 ou 6 atomes de carbone ou un radical aryle éventuellement substitué,
$R_2$ et $R_4$ identiques ou différents, représentent chacun un atome d'hydrogène ou le reste acyle d'un acide carboxylique aliphatique, cycloaliphatique ou aromatique,
m est égal à zéro ou un, et
X représente un atome d'hydrogène ou le groupement hydroxy-2 phényl-1 éthyle.

2. Composés selon la revendication 1, dans lesquels $R_F$ est un radical perfluoralkyle linéaire contenant de 5 à 15 atomes de carbone.

3. Composés selon la revendication 1 ou 2, dans lesquels :
- m est égal à 1 et $R_1$ et $R_2$ sont des atomes d'hydrogène ;
- m est égal à 0, $R_1$, $R_2$ et $R_4$ sont des atomes d'hydrogène et $R_3$ est un radical éthyle ; ou
- m est égal à 0, $R_1$, $R_2$ et $R_3$ sont des atomes d'hydrogène et $R_4$ est un radical benzoyle.

4. Mélanges de composés selon l'une des revendications 1 à 3, dans lesquels les groupements $R_F$ sont différents.

5. Mélanges, caractérisés en ce qu'ils contiennent un ou plusieurs composés selon l'une des revendications 1 à 3 et jusqu'à environ 80 % d'un ou plusieurs composés de formule:

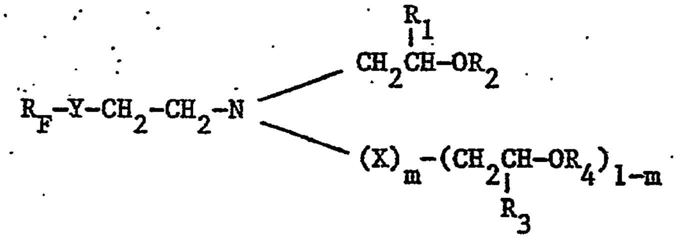 (IV)

dans laquelle les symboles $R_F$, m, X, $R_1$, $R_2$, $R_3$ et $R_4$ ont les mêmes significations que dans la revendication 1 et Y représente un radical $CF_2$ ou $CH_2$.

6. Procédé de préparation des produits fluorés selon l'une des revendications 1 à 5, caractérisé en ce que l'on soumet à une hydrogénation un amino-alcool de formule :

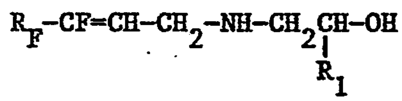 (V)

ou un mélange de ces amino-alcools entre eux et/ou avec jusqu'à environ 70 % d'amino-alcools saturés de formule :

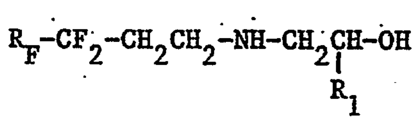 (IV-a)

et éventuellement, on soumet le produit obtenu à une époxydation et/ou à une estérification.

7. Utilisation des composés polyfluorés et des mélanges selon l'une des revendications 1 à 5 comme additifs anti-usure pour lubrifiants.

8. Lubrifiants, caractérisé en ce qu'ils contiennent un composé polyfluoré ou un mélange de composés polyfluorés selon l'une des revendications 1 à 5.

9. Lubrifiants selon la revendication 8, dans lesquels la teneur en composé(s) polyfluoré(s) est d'au moins 0,01 % en poids et, de préférence, comprise entre 0,02 et 0,5 %.

10. Lubrifiants selon la revendication 8 ou 9, dans laquels le ou les composés polyfluorés sont associés aux additifs classiques.

**Revendications pour l'Etat contractant: ES**

1. Procédé de préparation de composés polyfluorés répondant à la formule générale:

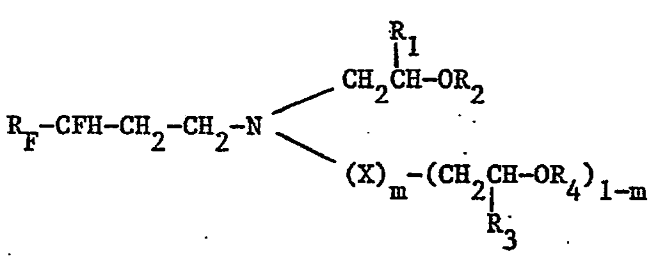 (III)

dans laquelle:

$R_F$ représente un radical perfluoroalkyle, linéaire ou ramifié, contenant de 1 à 19 atomes de carbone,

$R_1$ et $R_3$ identiques ou différents, représentent chacun un atome d'hydrogène, un radical alkyle contenant de 1 à 20 atomes de carbone, un radical cycloalkyle à 5 ou 6 atomes de carbone ou un radical aryle éventuellement substitué,

$R_2$ et $R_4$ identiques ou différents, représentent chacun un atome d'hydrogène ou le reste acyle d'un acide carboxylique aliphatique, cycloaliphatique ou aromatique,

m est égal à zéro ou un, et

X représente un atome d'hydrogène ou le groupement hydroxy-2 phényl-l éthyle,

ou de mélanges contenant un ou plusieurs de ces composés et jusqu'à environ 80% d'un ou plusieurs composés de formule:

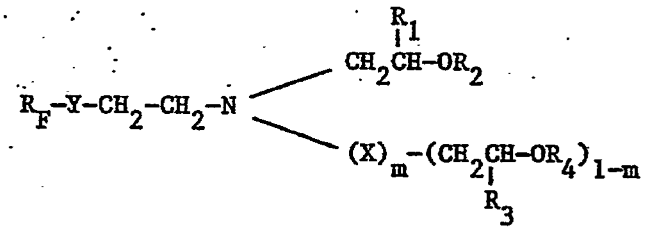 (IV)

dans laquelle les symboles $R_F$, m, X, $R_1$, $R_2$, $R_3$ et $R_4$ ont les mêmes significations que ci-dessus et Y représente un radical $CF_2$ ou $CH_2$, caractérisé en ce que l'on soumet à une hydrogénation un aminoalcool de formule:

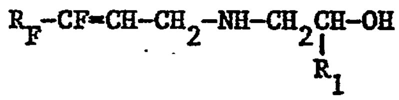 (V)

ou un mélange de ces amino-alcools entre eux et/ou avec jusqu'à environ 70% d'amino-alcools saturés de formule:

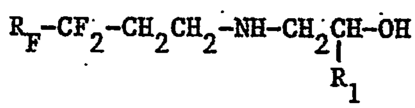 (IV-a)

et éventuellement, on soumet le produit obtenu à une époxydation et/ou à une estérification.

2. Procédé selon la revendication 1, dans lequel $R_F$ est un radical perfluoroalkyle linéaire contenant de 5 à 15 atomes de carbone.

3. Procédé selon la revendication 1 ou 2, dans lequel:
-m est égal à 1 et $R_1$ et $R_2$ sont des atomes d'hydrogène ;
-m est égal à 0, $R_1$, $R_2$ et $R_4$ sont des atomes d'hydrogène et $R_3$ est un radical éthyle; ou
-m est égal à 0, $R_1$, $R_2$ et $R_3$ sont des atomes d'hydrogène et $R_4$ est un radical benzoyle.

4. Utilisation des composés polyfluorés (III) et de leurs mélanges tels que définis dans la revendication 1, comme additifs anti-usure pour lubrifiants.

5. Utilisation selon la revendication 4, caractérisée en ce que la teneur du lubrifiant en composé(s) polyfluoré(s) est d'au moins 0,01% en poids et, de préférence, comprise entre 0,02 et 0,5%.

6. Utilisation selon la revendication 4 ou 5, caracterisée en ce que le ou les composés polyfluorés sont associés aux additifs classiques.

**Patentansprüche für die Vertragsstaaten: AT, BE, CH, DE, FR, GB, GR, IT, LI, LU, NL, SE**

1. Polyfluorierte Verbindungen, dadurch gekennzeichnet, daß sie der allgemeinen Formel

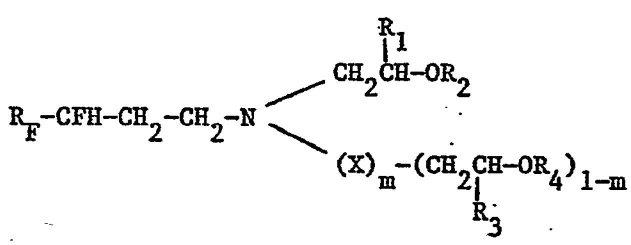 (III)

entsprechen, in der
$R_F$, einen geradkettigen oder verzweigten, 1 bis 19 Kohlenstoffatome enthaltenden Perfluoralkylrest bedeutet,
$R_1$ und $R_3$ identisch oder verschieden sind und jeder ein Wasserstoffatom, einen 1 bis 20 Kohlenstoffatome enthaltenden Alkylrest, einen 5 oder 6 Kohlenstoffatome enthaltenden Cycloalkylrest oder einen gegebenenfalls substituierten Arylrest bedeuten,
$R_2$ und $R_4$ identisch oder verschieden sind und jeder ein Wasserstoffatom oder den Acylrest einer aliphatischen, cycloaliphatischen oder aromatischen Carbonsäure bedeuten,
m gleich O oder 1 ist, und
X ein Wasserstoffatom oder die 2-Hydroxy-1-phenylethylgruppe bedeutet.

2. Verbindungen nach Anspruch 1, dadurch gekennzeichnet, daß $R_F$ ein geradkettiger, 5 bis 15 Kohlenstoffatome enthaltender Perfluoralkylrest ist.

3. Verbindungen nach Anspruch 1 oder 2, dadurch gekennzeichnet, daß
-m gleich 1 ist und $R_1$ und $R_2$ Wasserstoffatome sind;
-m gleich O ist, $R_1$, $R_2$ und $R_4$ Wasserstoffatome sind und $R_3$ ein Ethylrest ist; oder
-m gleich O ist, $R_1$, $R_2$ und $R_3$ Wasserstoffatome sind und $R_4$ ein Benzoylrest ist.

4. Gemische der Verbindungen nach einem der Ansprüche 1 bis 3, in denen die $R_F$- Gruppen verschieden sind.

5. Gemische, dadurch gekennzeichnet, daß sie eine oder mehrere Verbindungen nach einem der Ansprüche 1 bis 3 und bis zu etwa 80% einer oder mehrerer Verbindungen der Formel

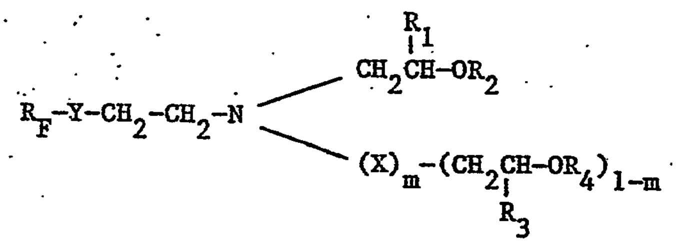 (IV)

enthalten, worin die Symbole $R_F$, m, X, $R_1$, $R_2$, $R_3$ und $R_4$ dieselben Bedeutungen wie in Anspruch 1 haben und Y einen $CF_2$- oder $CH_2$-Rest bedeutet.

6. Verfahren zur Herstellung von fluorhaltigen Produkten nach einem der Ansprüche 1 bis 5, dadurch gekennzeichnet, daß man einen Aminoalkohol der Formel

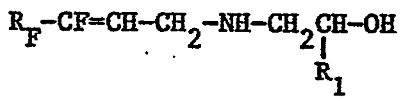 (V)

oder ein Gemisch dieser Aminoalkohole untereinander und/oder mit bis zu etwa 70% gesättigten Aminoalkoholen der Formel

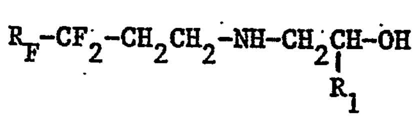 (IV-a)

einer Hydrierung unterzieht und gegebenenfalls das erhaltene Reaktionsprodukt einer Epoxydierung und/oder einer Veresterung unterwirft.

7. Verwendung der polyfluorierten Verbindungen und der Gemische nach einem der Ansprüche 1 bis 5 als Antiverschleißadditive für Schmiermittel.

8. Schmiermittel, dadurch gekennzeichnet, daß sie eine polyfluorierte Verbindung oder ein Gemisch aus polyfluorierten Verbindungen nach einem der Ansprüche 1 bis 5 enthalten.

9. Schmiermittel nach Anspruch 8, dadurch gekennzeichnet, daß der Gehalt an polyfluorierter(en) Verbindung(en) mindestens 0,01 Gew.% und vorzugsweise zwischen 0,02 und 0,5% beträgt.

10. Schmiermittel nach Anspruch 8 oder 9, dadurch gekennzeichnet, daß in ihnen die polyfluorierte(n) Verbindung(en) mit klassischen Additiven verbunden ist(sind).

**Patentansprüche für den Vertragsstaat: ES**

1. Verfahren zur Herstellung von polyfluorierten Verbindungen der allgemeinen Formel

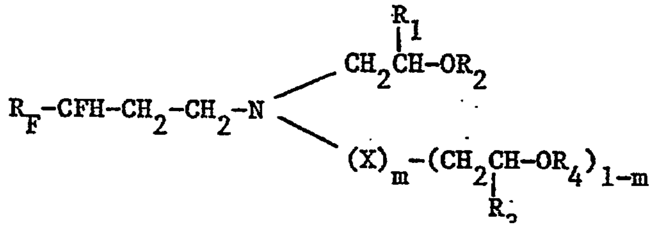 (III)

worin

$R_F$ einen geradkettigen oder verzweigten, 1 bis 19 Kohlenstoffatome enthaltenden Perfluoralkylrest bedeutet,
$R_1$ und $R_3$ identisch oder verschieden sind und jeder ein Wasserstoffatom, einen 1 bis 20 Kohlenstoffatome enthaltenden Alkylrest, einen 5 oder 6 Kohlenstoffatome enthaltenden Cycloalkylrest oder einen gegebenenfalls substituierten Arylrest bedeuten,
$R_2$ und $R_4$ identisch oder verschieden sind und jeder ein Wasserstoffatom oder den Acylrest einer aliphatischen, cycloaliphatischen oder aromatischen Carbonsäure bedeuten,
m gleich O oder 1 ist, und
X ein Wasserstoffatom oder die 2-Hydroxy-l-phenylethylgruppe bedeutet,
oder von Gemischen, die eine oder mehrere Verbindungen der
Formel (III) und bis zu etwa 80% einer oder mehrerer Verbindungen der Formel

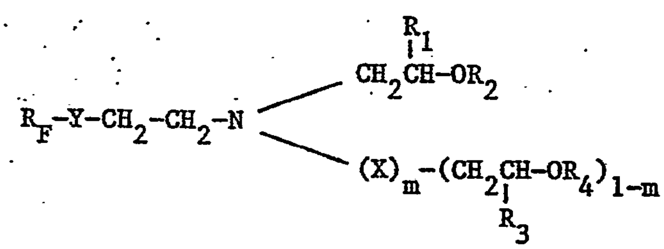
(IV)

enthalten, worin die Symbole $R_F$, m, X, $R_1$, $R_2$, $R_3$ und $R_4$ dieselben Bedeutungen wie oben haben und Y einen $CF_2$- oder $CH_2$-Rest bedeutet, dadurch gekennzeichnet, daß man einen Aminoalkohol der Formel

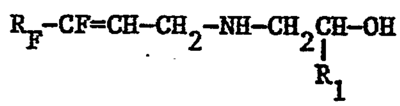
(V)

oder ein Gemisch dieser Aminoalkohole untereinander und/oder mit bis zu etwa 70% gesättigten Aminoalkoholen der Formel

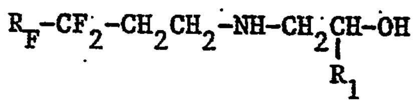
(IV-a)

einer Hydrierung unterzieht und gegebenenfalls das erhaltene Reaktionsprodukt einer Epoxydierung und/oder einer Veresterung unterwirft.

2. Verfahren nach Anspruch 1, dadurch gekennzeichnet, daß $R_F$ ein geradkettiger, 5 bis 15 Kohlenstoffatome enthaltender Perfluoralkylrest ist.

3. Verfahren nach Anspruch 1 oder 2, dadurch gekennzeichnet, daß
-m gleich 1 ist und $R_1$ und $R_2$ Wasserstoffatome sind;
-m gleich O ist, $R_1$, $R_2$ und $R_4$ Wasserstoffatome sind und $R_3$ ein Ethylrest ist; oder
-m gleich O ist, $R_1$, $R_2$ und $R_3$ Wasserstoffatome sind und $R_4$ ein Benzoylrest ist.

4 . Verwendung der polyfluorierten Verbindungen (III) und ihrer Gemische nach Anspruch 1, als Antiverschleißadditive für Schmiermittel.

5 . Verwendung nach Anspruch 4, dadurch gekennzeichnet, daß der Gehalt des Schmiermittels an polyfluorierter(en) Verbindung(en) mindestens 0,01 Gew.% und vorzugsweise zwischen 0,02 und 0,5% beträgt.

6. Verwendung nach Anspruch 4 oder 5, dadurch gekennzeichnet, daß die polyfluorierte(n) Verbindung(en) mit klassischen Additiven verbunden ist(sind).

**Claims for the Contracting States: AT, BE, CH, DE, FR, GB, GR, IT, LI, LU, NL, SE**

1. Polyfluoro compounds characterized in that they correspond to the general formula:

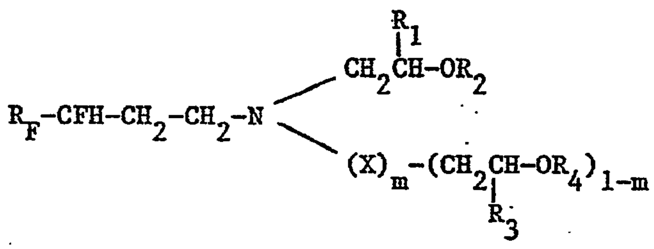

(III)

in which:
$R_F$ denotes a linear or branched perfluoroalkyl radical containing from 1 to 19 carbon atoms, each of $R_1$ and $R_3$, which are identical or different, denotes a hydrogen atom, an alkyl radical containing from 1 to 20 carbon atoms, a cycloalkyl radical containing 5 or 6 carbon atoms or an optionally substituted aryl radical, each of $R_2$ and $R_4$, which are identical or different, denotes a hydrogen atom or the acyl residue of an aliphatic, cycloaliphatic or aromatic carboxylic acid,
m is equal to zero or one, and
X denotes a hydrogen atom or the 2-hydroxy-1-phenylethyl group.

2. Compounds according to Claim 1, in which $R_F$ is a linear perfluoroalkyl radical containing from 5 to 15 carbon atoms.

3. Compounds according to Claim 1 or 2, in which:
-m is equal to 1 and $R_1$ and $R_2$ are hydrogen atoms;
-m is equal to 0, $R_1$, $R_2$ and $R_4$ are hydrogen atoms and $R_3$is an ethyl radical; or
-m is equal to 0, $R_1$, $R_2$ and $R_3$ are hydrogen atoms and $R_4$ is a benzoyl radical.

4. Mixtures of compounds according to one of Claims 1 to 3, in which the groups $R_F$ are different.

5. Mixtures characterized in that they contain one or more compounds according to one of Claims 1 to 3 and up to approximately 80% of one or more compounds of formula:

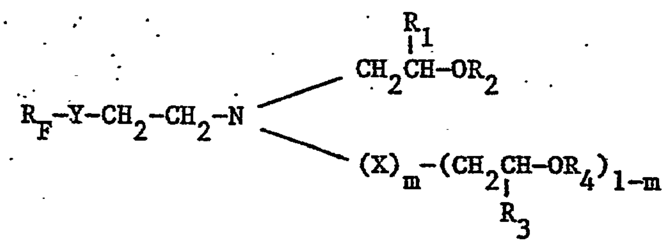

(IV)

in which the symbols $R_F$, m, X, $R_1$, $R_2$, $R_3$ and $R_4$ have the same meanings as in Claim 1 and Y denotes a $CF_2$ or $CH_2$ radical.

6. Process for the preparation of the fluorinated products according to one of Claims 1 to 5, characterized in that an aminoalcohol of formula:

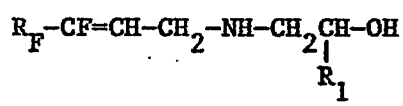

(V)

or a mixture of these aminoalcohols with each other and/or with up to approximately 70% of saturated aminoalcohols of formula:

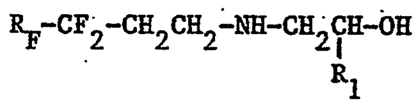

(IV-a)

is subjected to a hydrogenation and the product obtained is optionally subjected to an epoxidation and/or to an esterification.

7. Use of the polyfluoro compounds and of the mixtures according to one of Claims 1 to 5 as antiwear additives for lubricants.

8. Lubricants characterized in that they contain a polyfluoro compound or a mixture of polyfluoro compounds according to one of Claims 1 to 5.

9. Lubricants according to Claim 8, in which the content of polyfluoro compound(s) is at least 0.01% by weight and preferably between 0.02 and 0.5%.

10. Lubricants according to Claim 8 or 9, in which the polyfluoro compound(s) is (are) coupled with traditional additives.

**Claims for the Contracting State: ES**

1. Process for the preparation of polyfluoro compounds corresponding to the general formula:

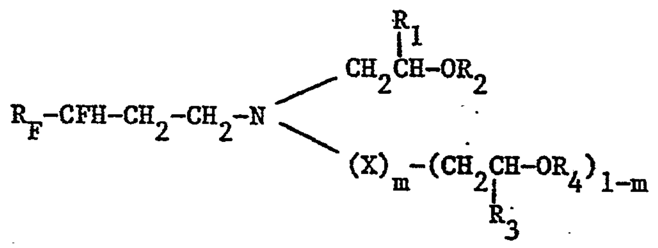

(III)

in which:

RF denotes a linear or branched perfluoroalkyl radical containing from 1 to 19 carbon atoms, each of $R_1$ and $R_3$, which are identical or different, denotes a hydrogen atom, an alkyl radical containing from 1 to 20 carbon atoms, a cycloalkyl radical containing 5 or 6 carbon atoms or an optionally substituted aryl radical, each of $R_2$ and $R_4$, which are identical or different, denotes a hydrogen atom or the acyl residue of an aliphatic, cycloaliphatic or aromatic carboxylic acid,

m is equal to zero or one, and

X denotes a hydrogen atom or the 2-hydroxy-1-phenylethyl group, or of mixtures containing one or more of these compounds and up to approximately 80% of one of more compounds of formula:

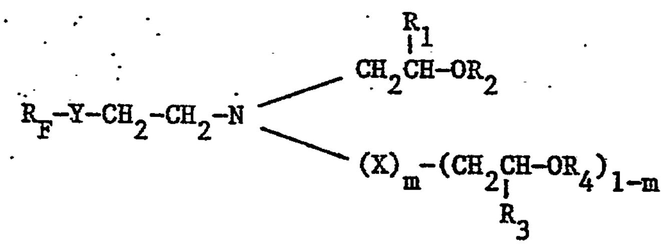

(IV)

in which the symbols $R_F$, m, X, $R_1$, $R_2$, $R_3$ and $R_4$ have the same meanings as above and Y denotes a $CF_2$ or $CH_2$ radical, characterized in that an aminoalcohol of formula:

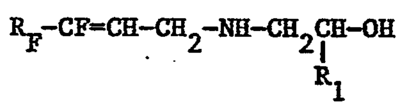

(V)

or a mixture of these aminoalcohols with each other and/or with up to approximately 70% of saturated aminoalcohols of formula:

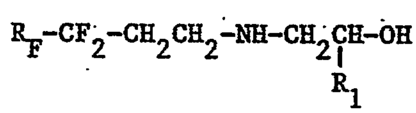

(IV-a)

is subjected to a hydrogenation and the product obtained is optionally subjected to an epoxidation and/or to an esterification.

2. Process according to Claim 1, in which $R_F$ is a linear perfluoroalkyl radical containing from 5 to 15 carbon atoms.

3. Process according to Claim 1 or 2, in which:

-m is equal to 1 and $R_1$ and $R_2$ are hydrogen atoms;

-m is equal to 0, $R_1$, $R_2$ and $R_4$ are hydrogen atoms and $R_3$ is an ethyl radical; or

-m is equal to 0, $R_1$, $R_2$ and $R_3$ are hydrogen atoms and $R_4$ is a benzoyl radical.

4. Use of the polyfluoro compounds (III) and of their mixtures such as defined in Claim 1, as antiwear additives for lubricants.

5. Use according to Claim 4, characterized in that the content of polyfluoro compound(s) in the lubricant is at least 0.01% by weight and preferably between 0.02 and 0.5%.

6. Use according to Claim 4 or 5, characterized in that the polyfluoro compound(s) is (are) coupled with traditional additives.